# EUROPEAN PATENT APPLICATION

(11) **EP 1 700 568 A2**
(43) Date of publication of application: **13.09.2006**
(21) Application number: 06251167.0
(22) Date of filing: 03.03.2006
(51) Int. Cl.: A61B 6/00, G01T 1/29, A61B 5/00

(54) **Breast cancer diagnostic apparatus for fused imaging of breast**

(30) Priority: 07.03.2005 US 74239
(71) Applicant: Fused Multi Modality Imaging Ltd, Solon , Ohio 44139 (US)
(72) Inventor: McCroskey, William K., Solon, Ohio 44139 (US); Dickinson, William D., Northfield Center Ohio 44067 (US); LeMaster, William S., Solon, Ohio 44139 (US); Summerhill, Walter A., Orwell, Ohio 44076 (US); Dobos, Alan M., Solon, Ohio 44139 (US); Milliff, Michael E., Chapin South Carolina 29036 (US)
(74) Representative: Gambell, Derek

(57) **Abstract**

A new method of breast imaging to improve the detection of cancer during early stages of development is disclosed. The system combines molecular images of radioisotope uptake in cancerous cells with three dimensional high resolution single photon emission computed tomography (SPECT), positron emission tomography (PET), x-ray computed tomography (CT) and optical reflectance and emission (ORE) images of the breast. The system acquires data from nuclear isotopes within the breast and processes the data into three dimensional molecular tomographic images of cancerous cellular activity, morphological three dimensional x-ray density tomographic images and three dimensional optical surface images. These three sets of images or data are then combined to provide information as to the sensitivity and specificity as to the type of cancer present, three dimensional information as to the physical location of the cancer and reference information for radiologists, surgeons, oncologists and patients in order to plan stereo-tactic biopsy, minimally invasive surgery and image guided therapy, if necessary.

## Description

### TECHNICAL FIELD

The present invention relates, in general, to gamma ray and x-ray detector systems and signal processing for nuclear medicine gamma cameras, single photon emission tomography (SPECT), positron emission tomography (PET), x-ray computed tomography (CT), digital radiology, x-ray mammography, optical imaging, optical fluorescence imaging, and other limited field of view gamma ray and x-ray detection and signal processing instrumentation.

### BACKGROUND ART

This invention applies to gamma ray imaging, nuclear SPECT imaging, PET imaging, x-ray CT imaging, digital radiography (DR) imaging, x-ray mammography, optical imaging, optical fluorescence imaging, small field of view imaging detectors and probes, and fused multimodality imaging.

In breast imaging and screening, x-ray mammography is being used as a screening tool for women over the age of 40 years. During the screening process, 40% of women have dense breast or suspicious breast indications for cancer. The radiologists reading these mammograms have difficulty reading the dense breast x-ray mammograms. A better method is needed for detecting cancer in dense breasts. Currently 8 out of 10 biopsies done on these patients indicate a false positive from x-ray mammography.

To improve the detection of breast cancer in women having dense breasts, a combination of molecular cellular functional images and x-ray density images of the breast is needed. Radioisotopes such as Tc-99m Sestamibi and positron isotopes such as FDG-F18 uptake in cancerous cells more rapidly than normal cells. Tc-99m Sestamibi molecules uptake in the mitochondria of the cell. Cancerous cells have more mitochondrial activity in comparison to normal surrounding cells. Similarly FDG F-18 uptake in cancerous cells is due to more glucose metabolism. The breast cancer cells uptake these isotopes more rapidly than the surrounding normal tissue. Thus, cancerous cells will emit more gamma rays as compared to normal cells.

In order to build a more sensitive and specific breast imaging device, the device must have higher spatial resolution and better contrast sensitivity than whole body imaging systems. Also the device must provide the location of the radioisotope distributions and anatomical x-ray density of breast tissues. In addition, the device must provide anatomical surface imaging of the breast superimposed with the radioisotope distributions and x-ray density of breast tissues and micro calcifications in three dimensions.

Today, projection x-ray mammography is used to detect breast density by compressing the breast tissue causing pain in some instances to the patient undergoing the mammographic exam. Once this exam has been completed and a dense breast indication has been found, there is not an easy alternative except to biopsy the breast tissues by surgery.

Scintigraphy has been used in conjunction with whole body gamma cameras with Tc-99m Sestamibi, but the sensitivity specificity drops below 40% when cancerous lesions are less than 2 cm in size. Ultrasound also may be used in the case of dense breasts but the procedure is very operator dependent. Therefore, there is a need for a more sensitive and specific breast imaging system which is comfortable for the patient and can provide true three dimensional information regarding potential breast cancer at the molecular level before anatomical changes occur. If there is a positive finding that breast cancer exists, then the system should provide three dimensional morphological information regarding the location of the cancer for surgical biopsy and rapid therapy.

### SUMMARY OF THE INVENTION

The present invention solves the problems that exist in prior art imaging systems and other problems by providing higher spatial resolution radioisotope imaging via breast anatomic specific imaging. The solution uniquely combines breast imaging with high resolution radioisotope imaging called micro single photon emission tomography (micro SPECT), high resolution positron emission tomography, micro positron emission tomography (micro PET), micro x-ray computed tomography (micro CT), and optical surface views. The term "micro" is used to describe the higher resolution capability of the system to image smaller details as compared to traditional whole body imaging, such as whole body gamma cameras, whole body PET scanners, and whole body CT scanners. The solution also allows the acquisition of breast information while the patient is lying prone and slightly tilted to one side and no contact is made with the breast during the imaging process. The solution provides anatomical and molecular images of the breast for detection of cancer and creates fused three dimensional images of the breast of anatomical x-ray density and molecular images of radioisotope uptake in breast tissues. The solution provides three dimensional information for stereo-tactic biopsy and breast surgery.

The present invention is directed to the basic building elements of modular curved radioisotope detection detectors for both single photon emitting isotopes and positron coincidence gamma ray emitting isotopes. The curved detectors are moved around the extended breast to collect data for micro SPECT and micro PET images. The unique scanning positions and oscillatory motion allow high resolution and high sensitivity detection of gamma rays emitted from respective isotopes. Also, x-ray micro CT images are generated from a focused modular breast curved x-ray detector array with micro collimated detection to reduce scattered radiation resulting in improved signal to noise images for low dose volume micro CT images. In addition, the upper outer quadrant of the breast can be imaged with a unique upper outer quadrant curved detector array oscillated and moved in a trajectory around the patient breast and axilla to produce tomographic images of the upper outer quadrant radioisotope distribution in both the upper outer quadrant (UOQ) micro SPECT mode and the UOQ micro PET mode.

Concurrent with radioisotope images, x-ray micro CT imaging can be produced of the central breast with a micro focused x-ray source and modular curved micro collimated detector array. The micro focused x-ray source and modular curved micro collimated detector array can be tiled and rotated to obtain micro CT of both the central breast and upper outer quadrant.

Concurrent with micro SPECT, micro PET, and micro X-ray CT modes, Optical Reflection and Emission (ORE) images representing surface views of the breast with multiple spectrums for indications of surface and near skin surface optical geometric and molecular information can be made. The Optical Reflection and Emission images are used for biopsy, interventional surgery in conjunction with fused molecular radioisotope images, and x-ray density images of the breast.

After the respective scans have been completed, the data are processed by unique tomographic breast reconstruction techniques and the respective sets of data are combined or fused together to show the cancerous tissues, if present, along with anatomical density images and optical surface views on a unique breast imaging workstation. If suspicious cancer areas are present, stereo-tactic biopsy, minimal invasive surgery, or image guided therapy can be planned and optimally conducted from the breast imaging workstation.

### BRIEF DESCRIPTION OF THE DRAWINGS

Figure 1 is a top frontal view of the apparatus utilized by the breast scan system of the present invention.
Figure 2 is a perspective view of the apparatus utilized by the breast scan system of the present invention showing the patient on a patient table.
Figure 3 is a system block diagram showing the architecture utilized by the breast scan system of the present invention.
Figure 4 is a perspective view of the apparatus utilized by the breast scan system of the present invention showing the patient tilted to one side on a patient table.
Figure 5 is a perspective view of a patient on a patient table and illustrates the upper outer quadrant gamma curved detector associated with the breast scan system of the present invention.
Figure 6 is an exploded view of the upper outer quadrant gamma curved detector shown in Figure 5.
Figure 7 is a top plan view of the upper outer quadrant gamma curved detector shown in Figures 5 and 6.
Figure 8 is a perspective view of the upper outer quadrant gamma curved detector, the central breast curved gamma detector, and the x-ray source and detector utilized by the breast scan system of the present invention.
Figure 9 is a front elevational view showing the position of the imaging components shown in Figure 8 with respect to a patient.
Figure 10 is a left end view showing the position of the imaging components shown in Figures 8 and 9 with respect to a patient.
Figure 11 is a front elevational exploded view of the upper outer quadrant gamma curved detector of the present invention and illustrates its position with respect to a patient.
Figure 12 is a left end exploded view of the upper outer quadrant gamma curved detector of the present invention and illustrates its position with respect to a patient at the beginning of a tomographic scan.
Figure 13 is a left end exploded view of the upper outer quadrant gamma curved detector of the present invention and illustrates its position with respect to the patient half way through a tomographic scan.
Figure 14 is a left end exploded view of the upper outer quadrant gamma curved detector of the present invention and illustrates its position with respect to the patient at the end of a tomographic scan.
Figure 15 is a perspective view of the central breast curved gamma detector, the central breast curved coincidence gamma detector, and the x-ray source and detector utilized by the breast scan system of the present invention.
Figure 16 is a left end view and a side view of the upper outer quadrant gamma curved detector and the central breast curved coincidence gamma detector of the breast scan system of the present invention.
Figure 17 illustrates micro PET imaging lines of response produced by the PET imaging components of the present invention.
Figure 18 is a perspective view of the single photon and coincidence gamma detector utilized by the breast scan system of the present invention.
Figure 19 is an end view of the single photon and coincidence gamma detector shown in Figure 18.
Figure 20 is a perspective view of the detector module utilized by the single photon and coincidence gamma detector shown in Figures 18 and 19.
Figure 21 is a front elevational view of the detector module shown in Figure 20 and a perspective view of the pixellated gamma detector elements contained therein.
Figure 22 is a front plan view of a patient showing a central breast scan and illustrating a representative position of the single photon and coincidence gamma detector utilized by the breast scan system of the present invention.
Figure 23 is an end view of a patient on a patient table showing an upper outer quadrant breast scan and a representative position of the single photon and coincidence gamma detector utilized by the breast scan system of the present invention.
Figure 24 is a front plan view of a patient showing an x-ray scan of the breast and representative positions of the x-ray source and detector during a scan.
Figure 25 is an end view showing breast scan data acquisition orbits and reconstruction of radioisotope distributions in a breast utilizing the breast scan system of the present invention.
Figure 26 is an end view showing breast scan data acquisition orbits and reconstruction of x-ray transmissions in a breast utilizing the breast scan system of the present invention.
Figure 27 is a schematic diagram showing the fusing of multimodality images by utilizing the breast scan system of the present invention.
Figure 28 is a front elevational view of a patient on a patient table and illustrates stereo-tactic biopsy, minimally invasive surgery, and image-guided therapy using multimodality images produced by the breast scan system of the present invention.

### DESCRIPTION OF THE PREFERRED EMBODIMENT

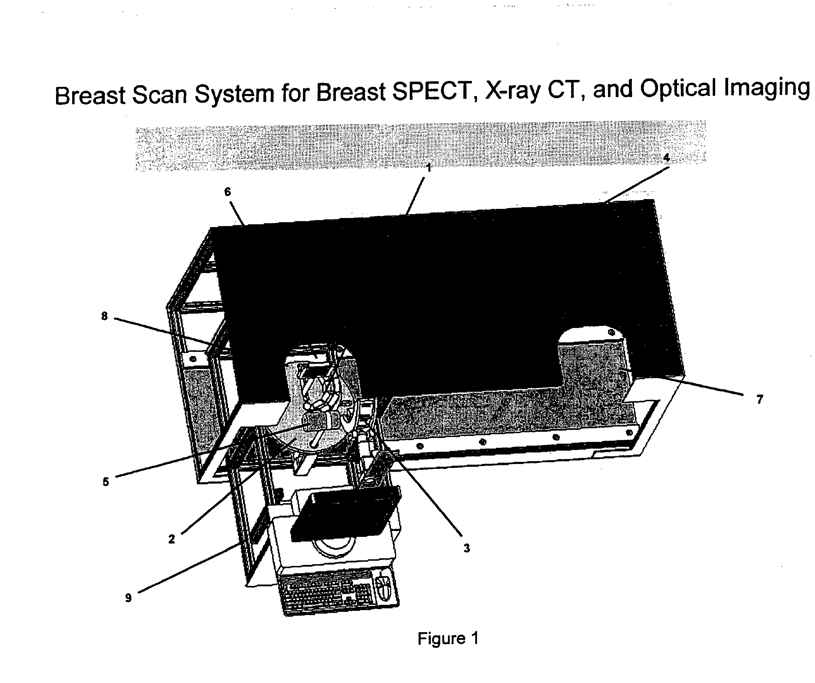

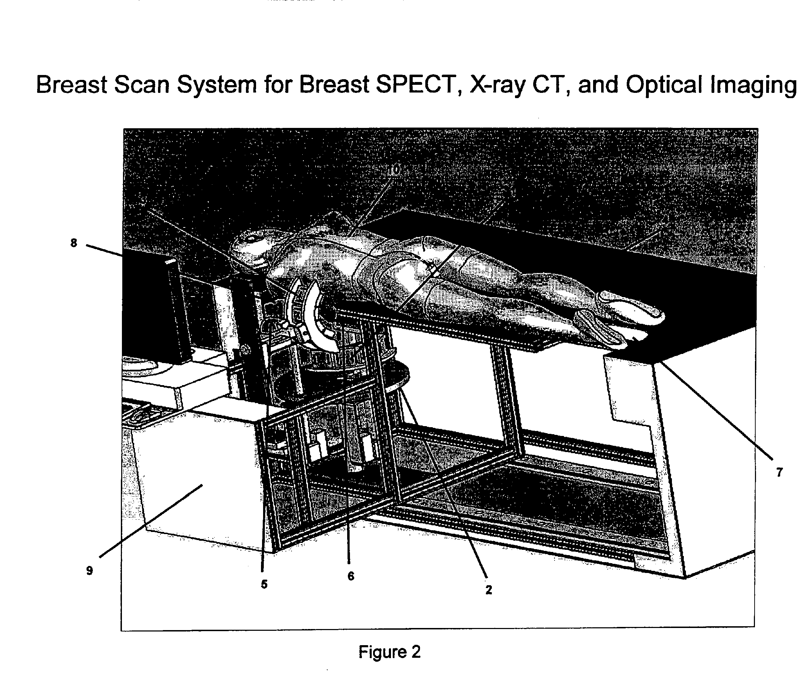

Referring now to the Figures where the illustrations are for the purpose of describing the preferred embodiment of the present invention and are not intended to limit the invention disclosed herein, Figure 1 is a top frontal view of the apparatus utilized by the breast scan system of the present invention. As shown in Figure 2, the patient 10 lies prone and slightly tilted to one side to allow full extension of the breast through a left breast hole 8 or right breast hole 7. The breast is scanned with an anatomic specific imaging central breast curved gamma detector 1 for single photon emission computed tomography (SPECT). Radioisotopes are injected into the patient 10 and emitted radiation is detected by the central breast curved gamma detector 1. The breast scan system also has an x-ray source 5 and an x-ray detector 6. The x-ray source 5 transmits x-rays through the breast of the patient 10 which are detected by the x-ray detector 6. The x-ray source 5 and x-ray detector 6 are rotated around the patient's breast on a rotate table 2. Also the central breast curved gamma detector 1 is rotated around the patient's breast on rotate table 2.

The upper outer quadrant gamma curved detector 3 can be positioned to image the upper outer quadrant of the breast to the axilla. The upper outer quadrant gamma curved detector 3 collects radioisotope information from the patient's breast area where the central breast curved gamma detector 1 cannot be positioned. The sliding detector carriage 9 allows the imaging components to be translated horizontally from the left breast hole 8 or to the right breast hole 7, and vice versa, to image the respective breast.

In Figure 2, the patient 10 is shown lying prone and slightly tilted to one side on breast imaging patient table 4 and over left breast hole 8. The patient's breast is extended by gravity for imaging. The patient is injected with a radioisotope which accumulates in cancerous tissues of the breast more rapidly than normal tissues. The central breast curved gamma detector 1 detects gamma rays emitted from the radioisotope distributions. The central breast curved gamma detector 1 is designed to anatomical fit close to the shape of the central breast to collect gamma rays being emitted. The central breast curved gamma detector 1 is rotated around the patient's central breast by rotate table 2. The upper outer quadrant gamma curved detector 3 is positioned around the patient's thorax to collect gamma rays from the upper outer quadrant of the breast to the axilla. The breast anatomy is a complex imaging area and the system is designed to image the entire breast including the lymph nodes. The upper outer quadrant gamma curved detector 3 can be positioned three dimensionally around the patient's thorax with vertical, horizontal, traverse, and oscillations to collect data while being very close to the patient 10.

As shown, x-ray source 5 and x-ray detector 6 are mounted to the rotate table 2. This allows for x-ray micro computed tomography of the breast. The x-ray source 5, x-ray detector 6, and central breast curved gamma detector 1 are all positioned around the patient's breast on the rotate table 2 to acquire high resolution single photon emission computed tomographic (SPECT) images and x-ray high resolution computed tomography (CT) images of the breast. In addition, the sliding detector carriage 9 allows imaging of the left breast through the left breast hole 8 and then translates to right breast hole 7 for repositioning of the patient for right breast imaging.

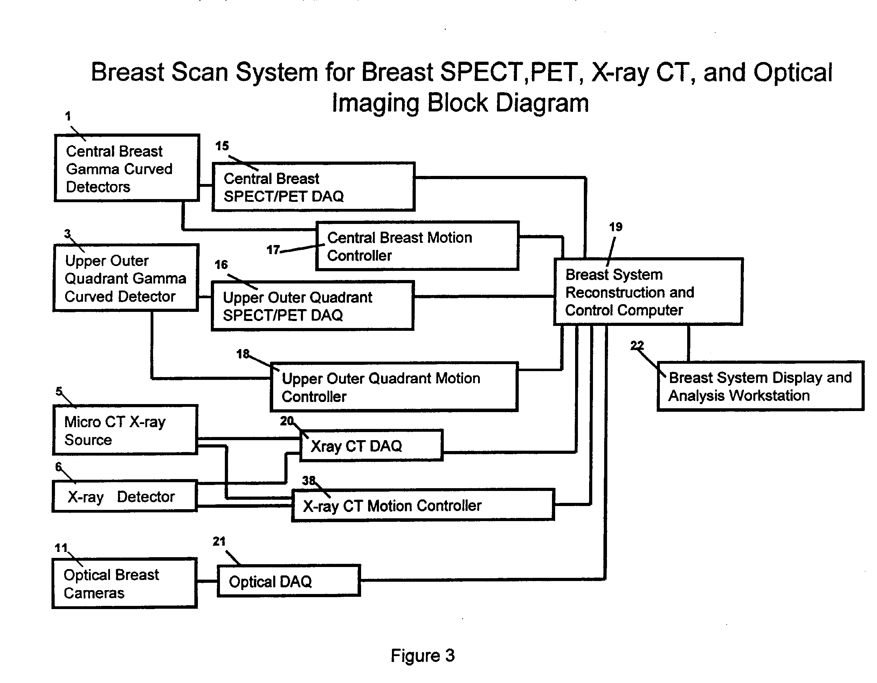

Referring now to Figure 3, the overall architecture and system structure is shown. Gamma rays are detected by either the central breast gamma curved detector(s) 1 and/or the upper outer quadrant gamma curved detector 3. These detectors can collect gamma rays emitted from single photon emitting isotopes, such as Tc-99m, or positron emitting isotopes, such as F-18. When using the positron emitting isotopes, coincidence detection is used to collect and determine the angle of the pair of 180 degree opposed gamma rays emitted from a positron annihilation. The central breast SPECT/PET DAQ block 15 controls and acquires both single photon gamma rays and coincidence gamma rays from the central breast gamma curved detectors 1 to form isotope projection images. The central breast motion controller 17 controls the geometric positioning of the central breast gamma curved detectors 1 including, rotation, vertical, radial, oscillate, and tilt positioning. The upper outer quadrant SPECT/PET DAQ block 16 controls and acquires both single photon gamma rays and coincidence gamma rays from upper outer quadrant curved gamma detector 3 to form isotope projection images. The upper outer quadrant motion controller 18 controls the geometric positioning of the upper outer quadrant gamma curved detector 3 including rotation, vertical, radial, oscillate, and tilt positioning.

As shown, x-ray CT DAQ 20 interfaces with the micro CT x-ray source 5 and x-ray detector 6 to acquire projection x-ray images through the breast anatomy. The micro CT x-ray source 5 and x-ray detector 6 are positioned by the x-ray CT motion controller 38 for x-ray micro CT of breast densities. The x-ray CT DAQ block 20 controls and acquires data from the micro CT x-ray source 5 and the x-ray detector 6. The x-ray CT DAQ 20 controls the x-ray detector 6 to generate projection views through the breast anatomy and form two dimension frames of attenuated x-rays. For optical images of the breast, optical breast cameras 11 are attached to respective micro CT x-ray source 5, x-ray detector 6, central breast gamma curved detectors 1, and upper outer quadrant gamma curved detector 3. The optical DAQ 21 controls the optical breast cameras 11 to generate optical views of the breast for spectral image of the breast at various wavelengths. The breast system reconstruction and control computer 19 controls and collects data from respective data acquisition (DAQ) and motion controllers. Specifically, the projection gamma images, coincidence gamma images or positron emission tomography (PET) images, x-ray projection images, and optical images are processed by the breast reconstruction and control computer 19 to form micro SPECT volumes, micro PET volumes, micro CT volumes of the breast anatomical density and radioactive isotope uptake in breast tissues. Also the breast reconstruction and control computer 19 geometrically overlays the optical views of the breast in co-registration with micro SPECT, micro PET, and micro CT three dimensional information. The three dimensional breast data from the respective modalities of micro SPECT, micro PET, micro CT, and optical surface image spectrums are combined together or fused on the breast display and analysis workstation 22.

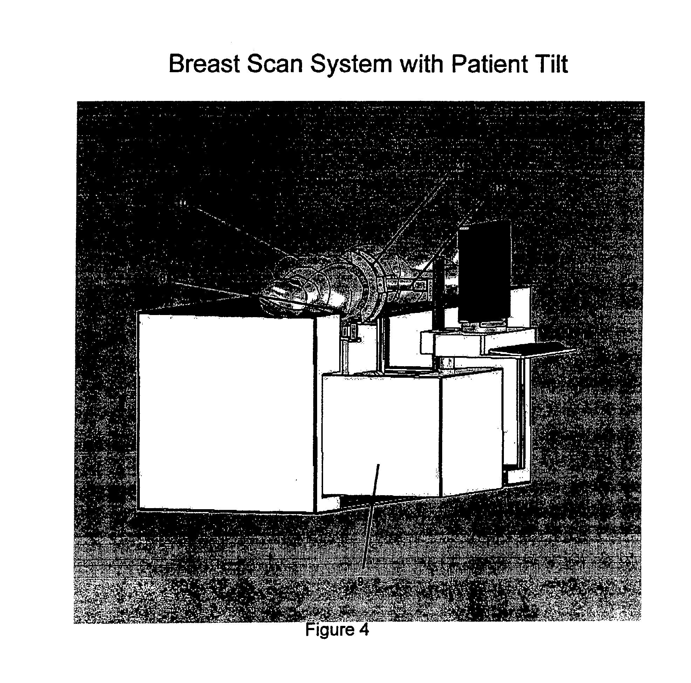

Referring now to Figure 4, the patient 10 lies on the patient table slightly tilted to one side to allow full breast extension by gravity into the left breast hole 8. The sliding detector carriage 9 can be positioned interactively by an operator for alignment on the center of the left breast. The scans can then be done on the left breast. Also shown is the upper outer quadrant gamma curved detector 3 which can be positioned to image the upper outer quadrant of the breast. The upper outer quadrant gamma curved detector 3 can be positioned by the upper outer quadrant motion controller 18 in an elliptical and oscillatory motion to obtain enough views to tomographically reconstruct the upper outer quadrant region of the breast.

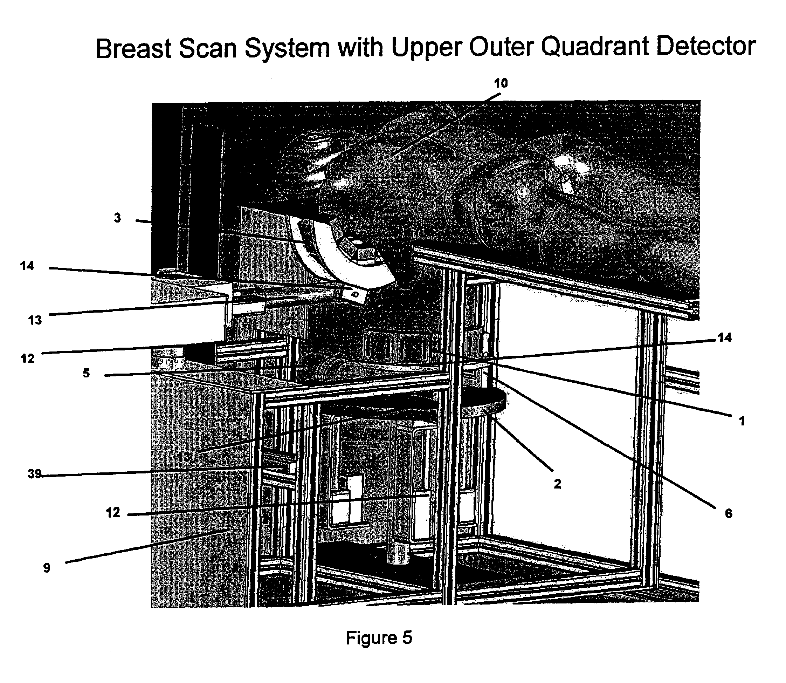

In Figure 5, the patient 10 is shown lying prone and slightly tilted to one side with her left breast extended into the left breast hole. The central breast curved gamma detector 1 is shown mounted to an oscillate positioner 14, a vertical positioner 12, radial positioner 13, rotate table 2, and to the sliding detector carriage 9. The x-ray source 5 and x-ray detector 6 are also maneuvered about the patient's breast with their respective vertical positioners on rotate table 2. The upper outer quadrant gamma curved detector 3 is positioned around the patient's breast and thorax. The upper outer quadrant gamma curved detector 3 is maneuvered with its respective oscillate positioner 14, radial positioner 13, vertical positioner 12, traverse positioner 39, and sliding detector carriage 9.

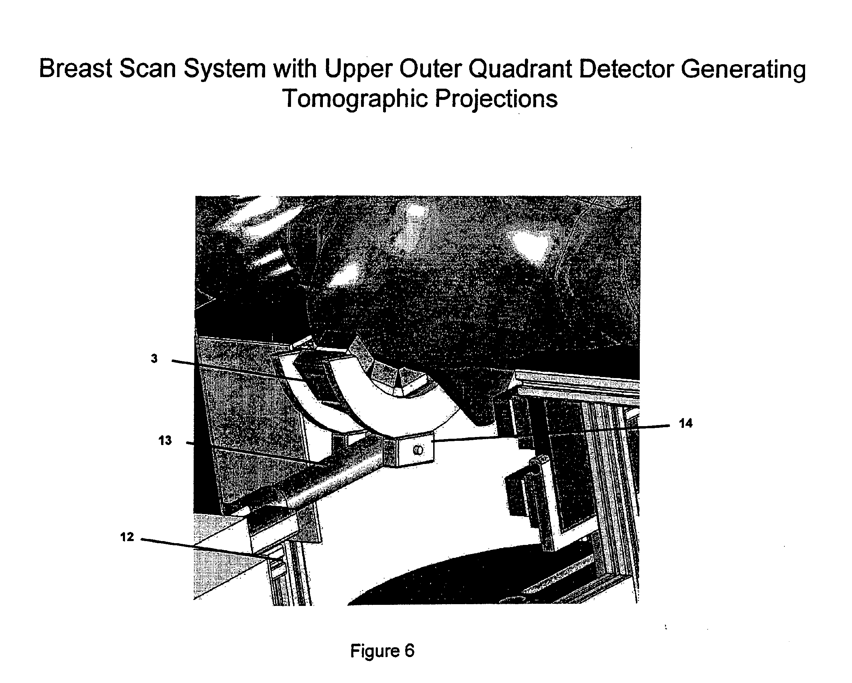

Referring now to Figure 6, the upper outer quadrant gamma curved detector 3 is shown close to the patient's chest and upper outer quadrant of the patient's breast. The upper outer quadrant gamma curved detector 3 is positioned close to the patient's breast anatomy via oscillate positioner 14, radial positioner 13, vertical positioner 12, and transverse positioner 39 mounted on sliding detector carriage 9.

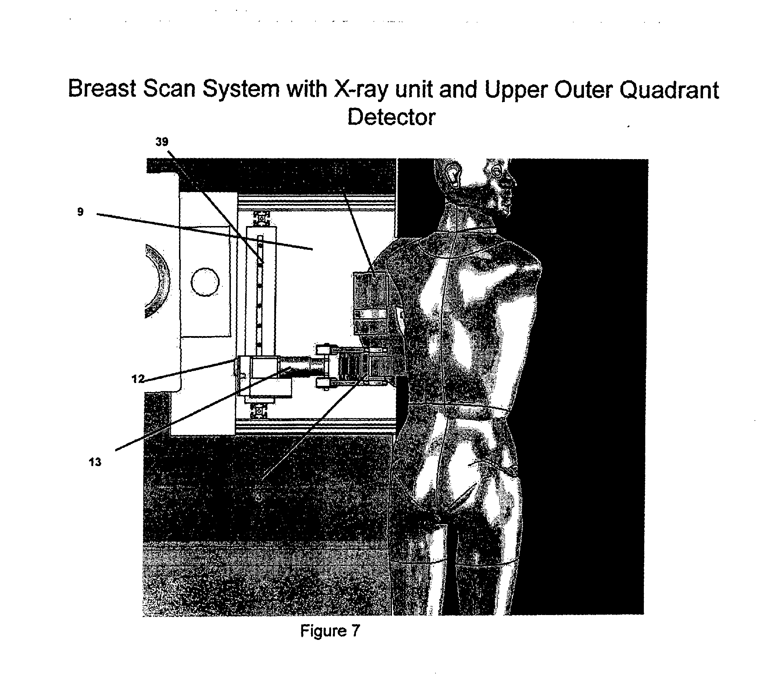

In Figure 7, the upper outer quadrant gamma curved detector 3 is shown being positioned with coordinated motion via oscillate positioner 14, radial positioner 13, vertical positioner 12, and transverse positioner 39 mounted on sliding detector carriage 9.

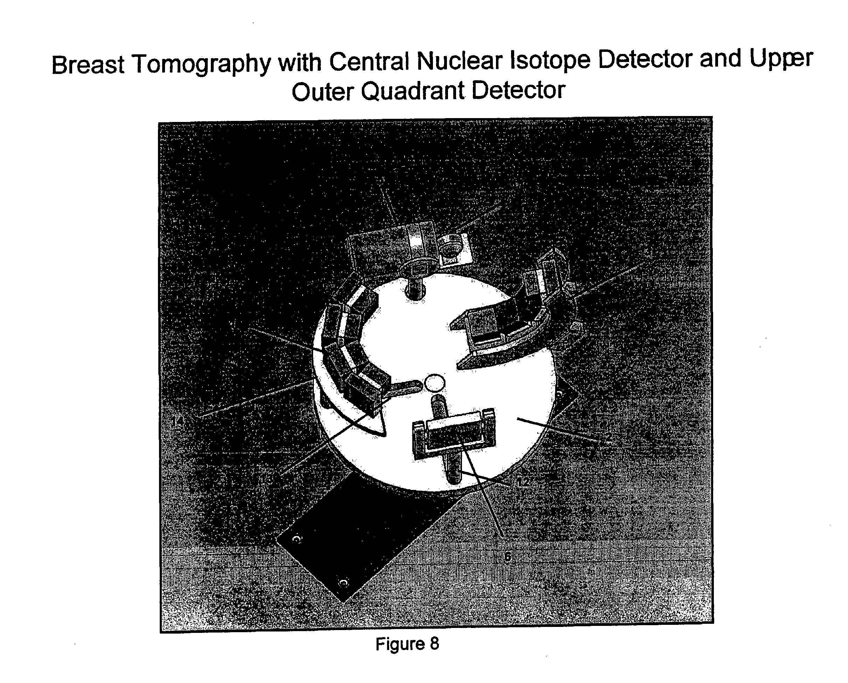

As shown in Figure 8, the apparatus utilized to obtain multiple angular radioisotopes views, x-ray views, and optical spectrum views of the breast is illustrated. For the central breast scan, the central breast curved gamma detector 1, x-ray source 5 and x-ray detector 6 are rotated around the breast on rotate table 2. The central breast curved gamma detector 1 x-ray source 5 and x-ray detector 6 have a respective oscillate positioner 14, vertical positioner 12, and radial positioner 13 to be moved around the central breast in a coordinated motion to collect anatomic specific views. The position orbits and respective oscillations of respective components allow the central breast curved gamma detector 1 to be positioned close to the breast without touching the breast to improve spatial resolution of and sensitivity to radioisotope distributions within the breast. Also geometric and temporal x-ray views of the breast can be done with x-ray source 5 and x-ray detector 6 being positioned via their respective vertical positioners 12, radial positioners 13, and rotate table 2. The position of the upper outer quadrant gamma curved detector 3 can be synchronized with central breast imaging components.

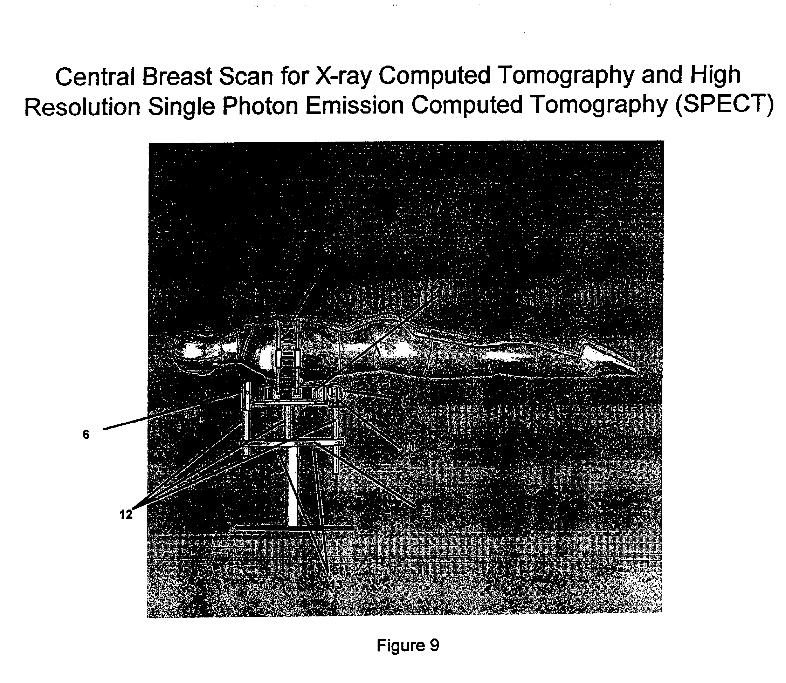

Referring now to Figure 9, the system concept is shown from a side view with the patient 10 lying prone and slightly tiled to one side with full breast extension by gravity. The x-ray source 5 and x-ray detector 6 are shown with their respective vertical positioners 12 and rotate table 2.

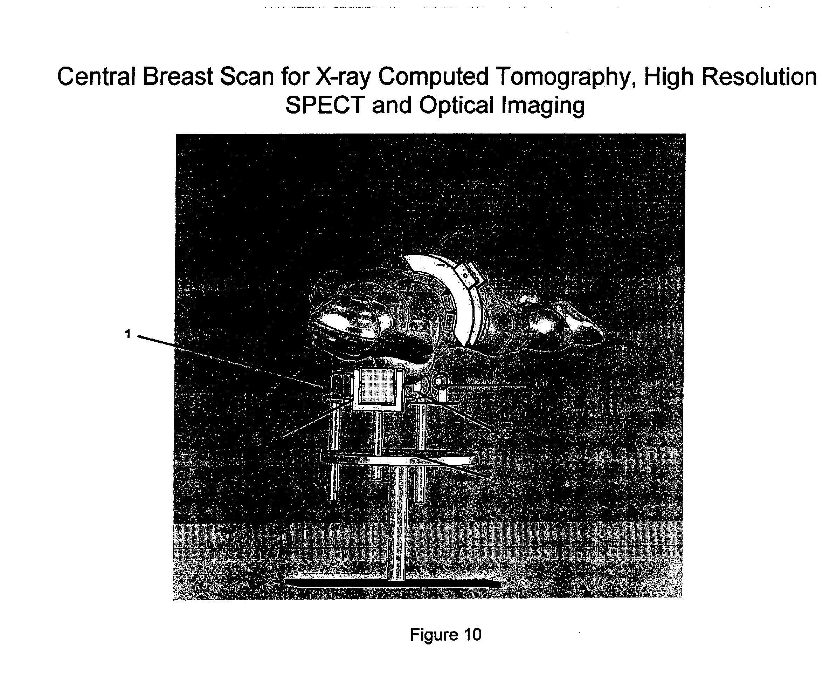

In Figure 10, the central breast curved gamma detector 1 is shown collecting projection view data of radioisotope distributions while being positioned close to the breast anatomy. Also the x-ray source 5 and x-ray detector 6 are also positioned on common rotate table 2. An optical breast camera 11 is shown to take temporally synchronized views of the breast's optical reflections, transmissions, and fluorescence at various spectrums or wavelengths. One use of the optical views is for breast surface registration with respective x-ray transmission and radioisotope views.

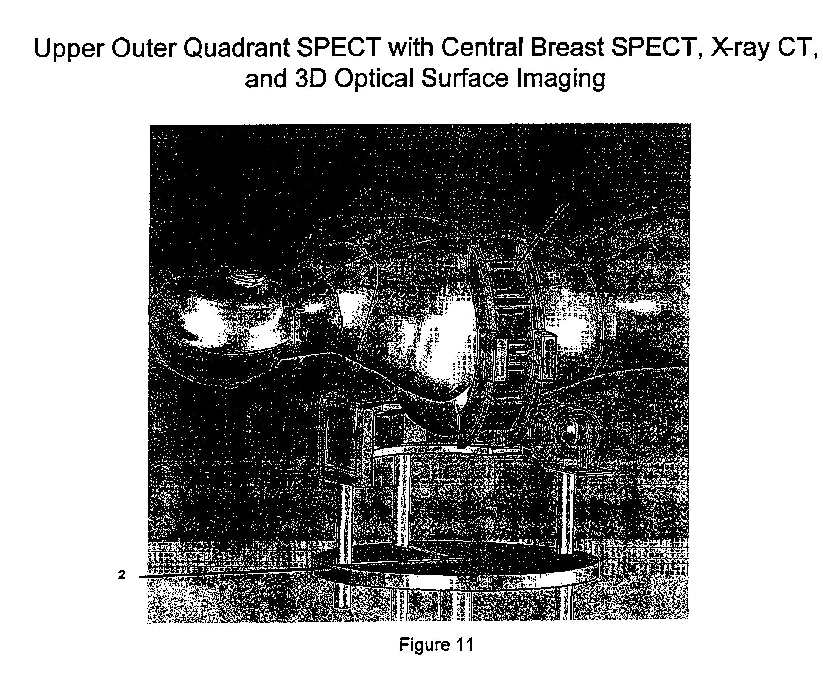

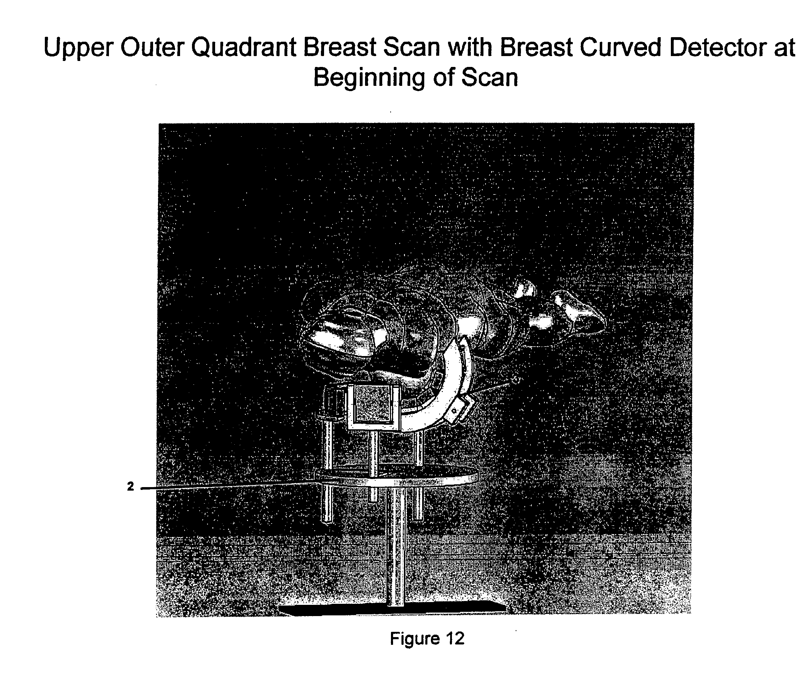

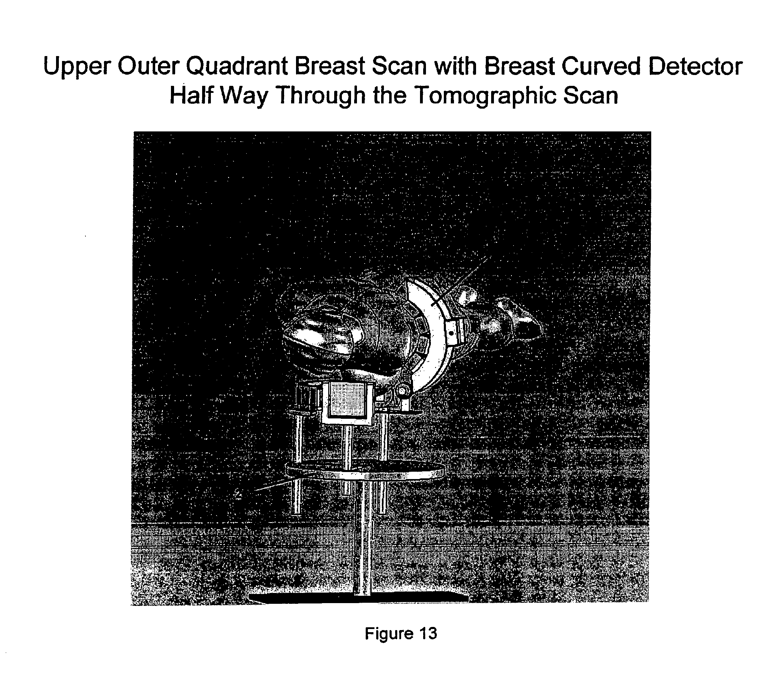

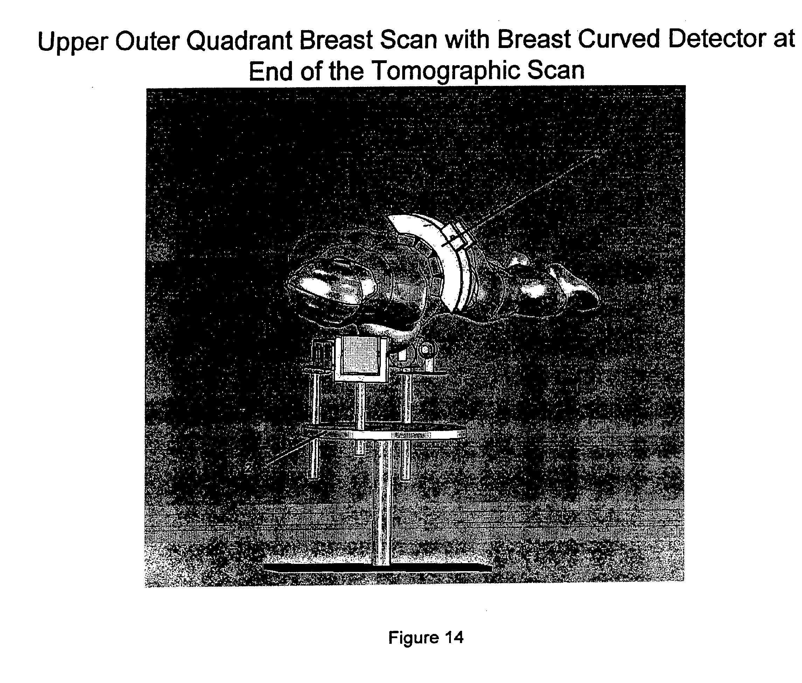

Referring now to Figures 11, 12, 13, 14, various positions of the upper outer quadrant gamma curved detector 3 are shown collecting gamma rays from radioisotope distributions within the breast and lymph nodes located close to the breast. The upper outer quadrant area of the breast is the location where 50% of cancers occur. Figure 14 shows views from the back and left side of patient; Figure 11 from the left side of patient and breast; Figure 12 from the left front side of chest wall and breast; and Figure 13 from the left back side of chest wall and breast.

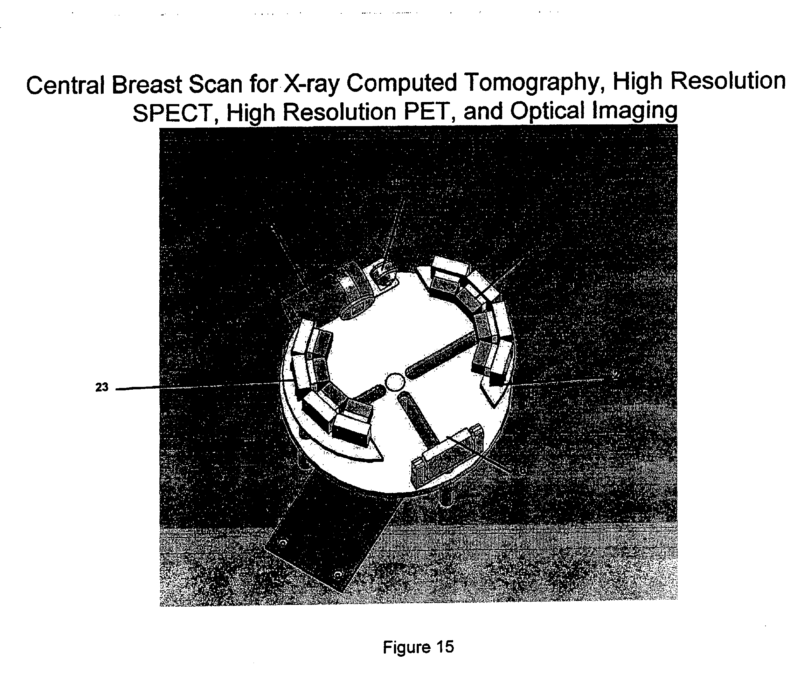

In Figure 15, the central breast curved coincidence gamma detector 23 is shown to allow coincidence detection of positron emitting isotopes, like F-18. The central breast curved gamma detector 1 and central breast curved coincidence gamma detector 23 are operated with temporal coincidence window between each event collected on the respective detector to form a line of response (LOR) between detector elements. The central breast curved coincidence gamma detector 23 is also rotated on rotate table 2 and can be positioned with its respective positioners. Also, the central breast curved coincidence gamma detector 23 can be used for single photon gamma detection and work in concert with central breast curved gamma detector 1 to form SPECT image projections improving sensitivity and specificity of the imaging system.

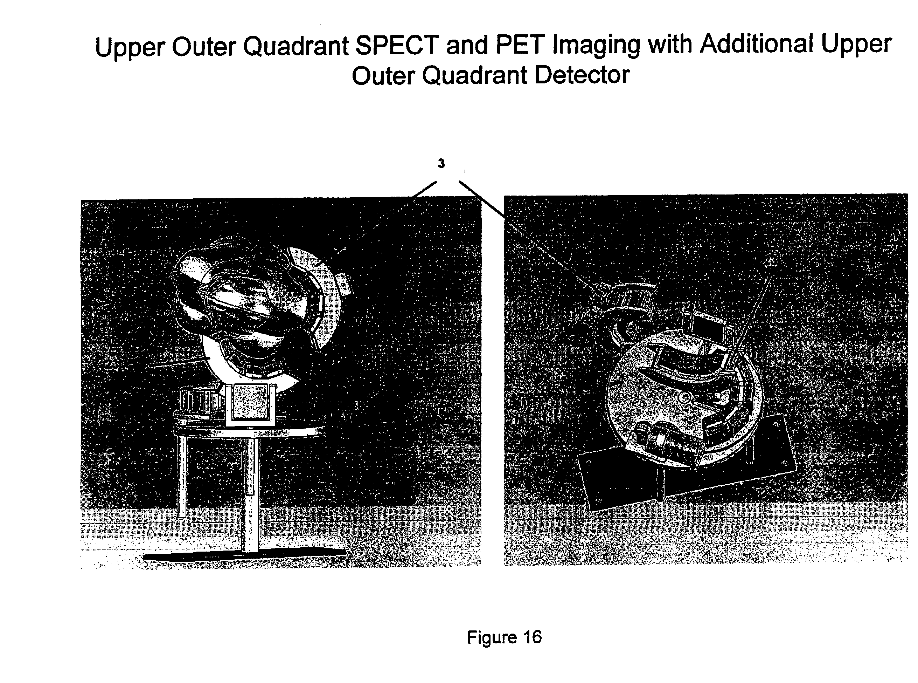

Referring now to Figure 16, the central breast curved coincidence gamma detector 23 may be used to operate in coincidence with the upper outer quadrant gamma curved detector 3. This allows for positron imaging of the upper outer quadrant for detection of cancer and lymph node uptake of radioisotope.

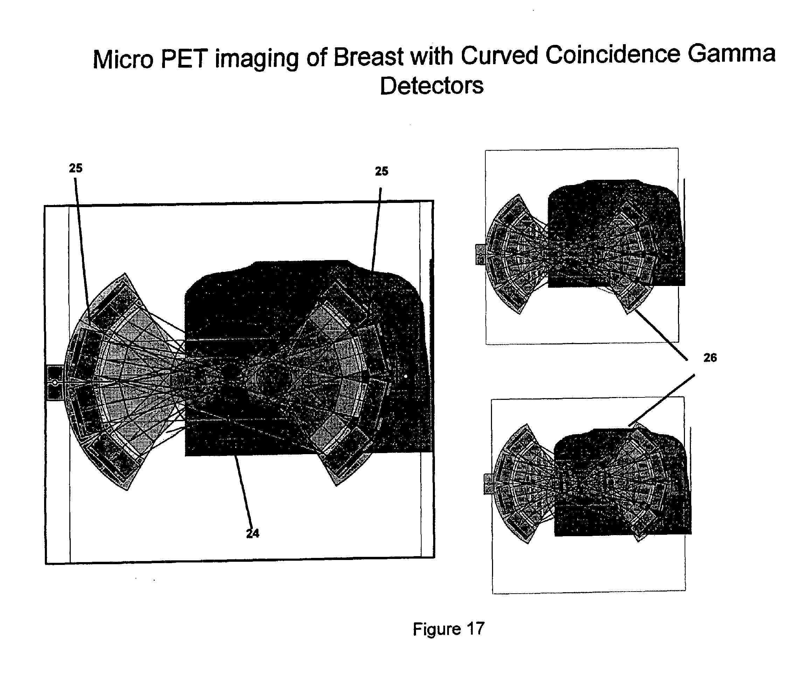

In Figure 17, the coincidence lines of response 24 are shown between respective breast curved single photon and coincidence gamma detectors 25. Also the entire breast volume can be imaged with translation, rotation, oscillating curved gamma detector motion 26.

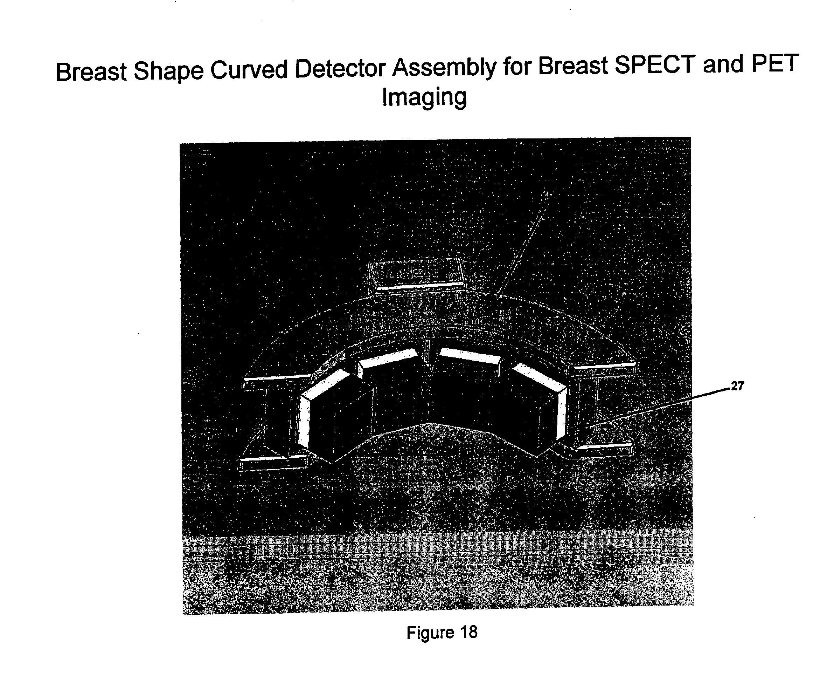

As shown in Figure 18, the breast curved single photon and coincidence gamma detector 25 is comprised of breast curved single photon and coincidence gamma detector module(s) 27. The modules 27 are mounted to form an anatomic breast shaped curved detector. The breast curved single photon and coincidence gamma detector module 27 can efficiently image lower energy single photon emitting isotopes, such as Tc-99m, at 140.5 Kev as well as 511Kev coincidence gamma rays from positron emitters, such as F-18. When imaging positron emitters, two breast curved single photon and coincidence gamma detectors 25 are operated in coincidence mode facing each other, as shown in Figure 17.

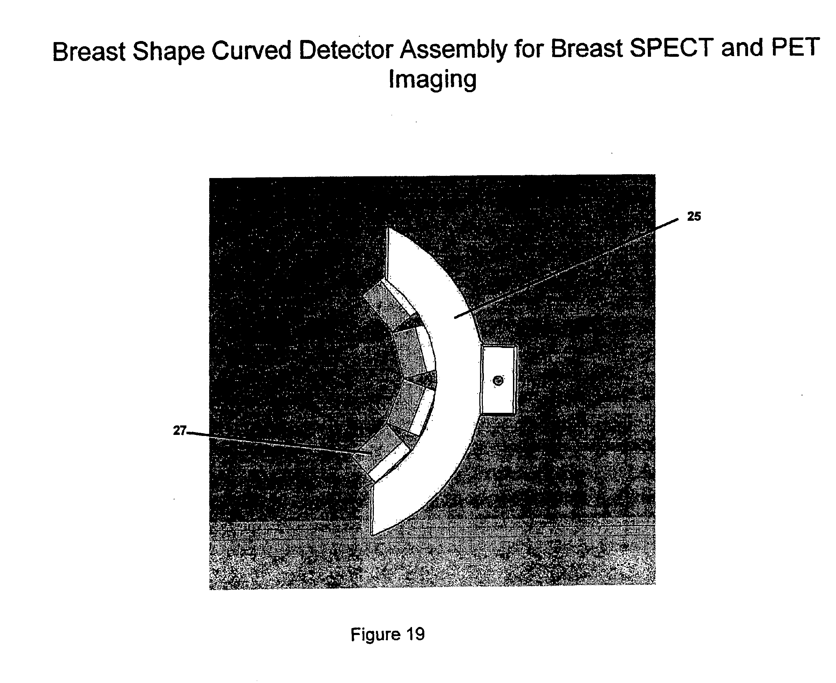

Referring now to Figure 19, the breast curved single photon and coincidence gamma detector 25 is shown and includes a plurality of multiple breast curved single photon and coincidence gamma detector modules 27.

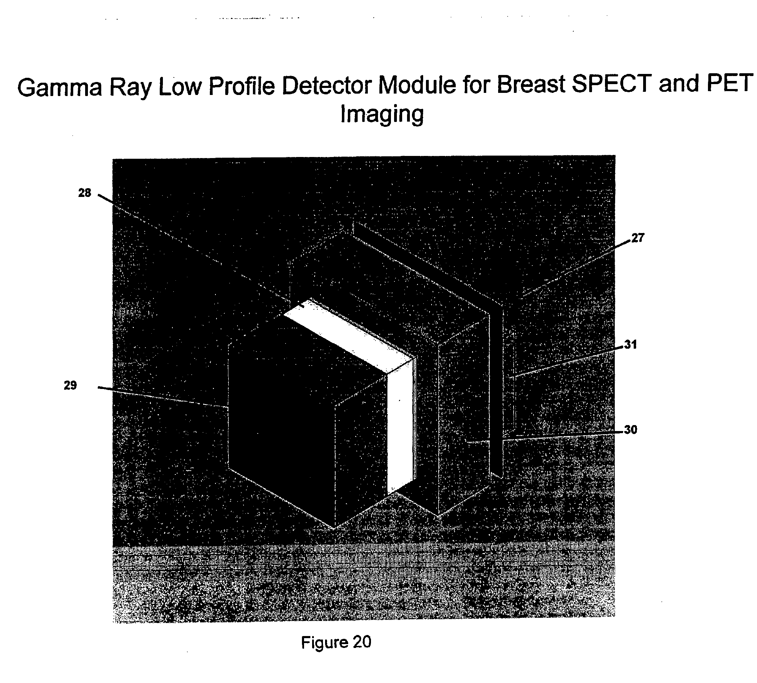

In Figure 20, the major components of the breast curved single photon and coincidence gamma detector module 27 are shown. Gamma rays and x-rays enter the module 27 via gamma and coincidence collimaor 29. The collimator mechanically focuses gamma rays for a common set of angles. In the preferred embodiment, parallel hole collimation is used to allow imaging of single photon emitting radioisotopes. The collimation provides the spatial resolution for SPECT imaging. In 511 Kev positron gamma ray imaging, the collimation acts as an anti-scatter grid to reduce down-scatter radiation from 511 Kev interaction in patient. The collimation is designed with high resolution parameters and along with positioning of the detector closer to patient provides greatly improved spatial resolution and isotope sensitivity. Pixelated gamma detector elements 28 or pixilated scintillation crystals are used to provide high resolution images. The pixelated array is interposed between the gamma and coincidence collimation 29 and low profile micro channel amplifier 30. The pixelated gamma detector elements 28 convert gamma rays into visible light. The low profile micro channel amplifier 30 converts the light to electrons that are amplified. The single and coincident gamma DAQ electronics 31 convert the amplified electrons from the low profile micro channel amplifier 30 to digital signals representing geometric position, energy level, and time of gamma event interaction with breast curved single photon and coincidence detector module.

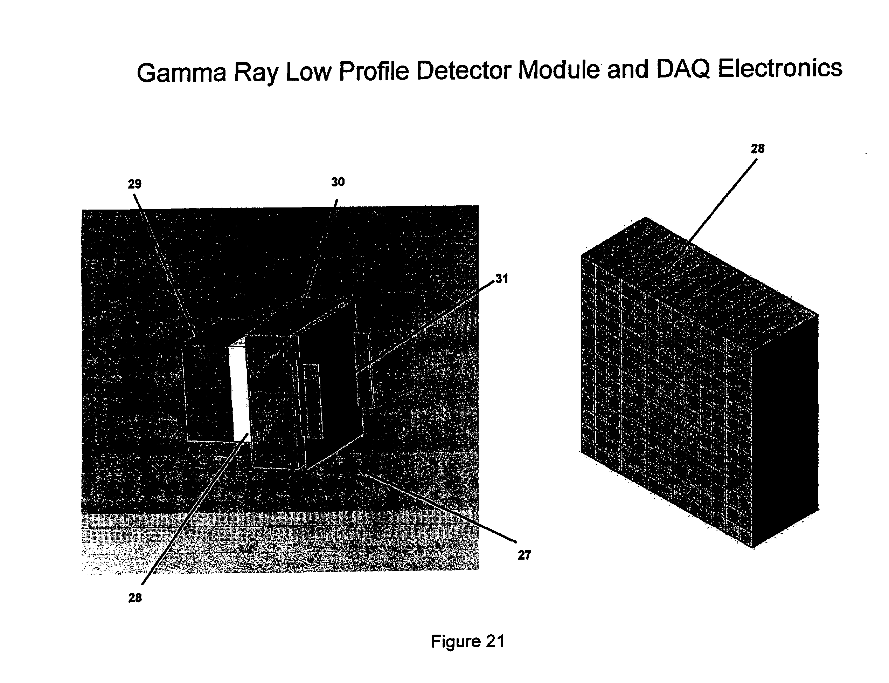

As shown in Figure 21, the pixelated gamma detector elements 28 are illustrated and a side view of the breast curved single photon and coincidence gamma detector module 27 are shown. The pixelated gamma detector elements 28 channel the scintillation light down independent channels and allow for high count rate data acquisition with multiple events occurring within the pixelated array. The septa between the respective pixels is designed to allow shaping of light distributions for high spatial and energy resolution of events in pixels with adaptive weighted positioning algorithms in the single and coincident gamma DAQ electronics 31.

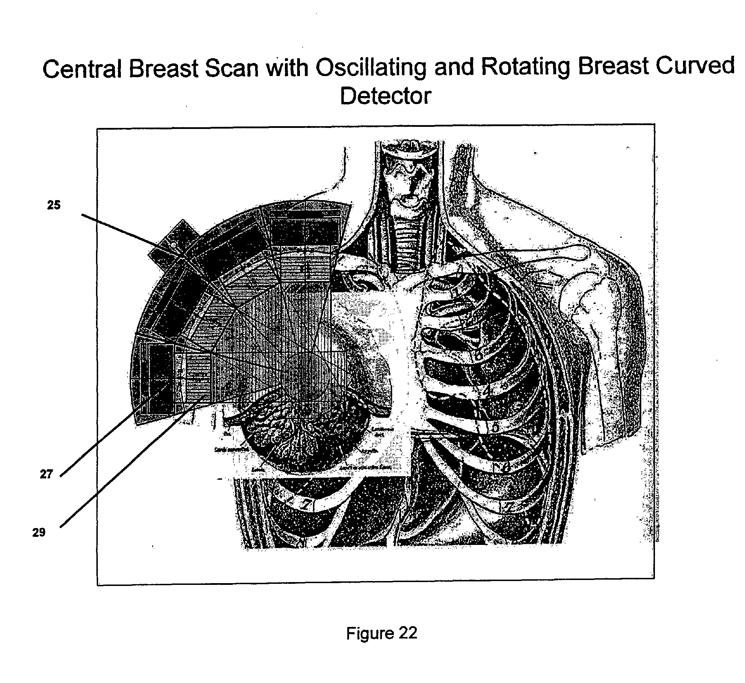

Referring now to Figure 22, the breast curved single photon and coincidence gamma detector 25 is shown positioned close to the central breast anatomy allowing for generation of tomographic views of the breast. The breast single photon and coincidence gamma detector modules 27 are placed in a curved configuration to allow close view of the breast without touching the breast. The breast curved single photon and coincidence gamma detector 25 can be geometrically maneuvered by positioners and motion control systems. Also shown is a focused collimation system 29 to view radioisotope distributions

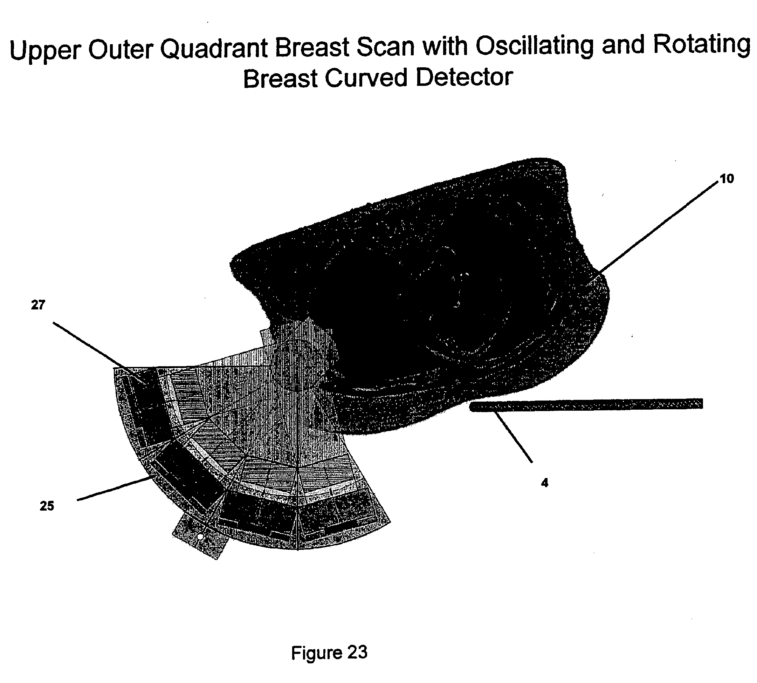

In Figure 23, the breast curved single photon and coincidence gamma detector 25 is shown generating views of the upper outer quadrant of the patient's breast. Each of the breast single photon and coincidence detector modules 27 provides a tomographic view with unique rotation and oscillation about the outer side of the patient's breast, chest and back while the patient 10 is lying prone on patient table 4 with breast extended via gravity.

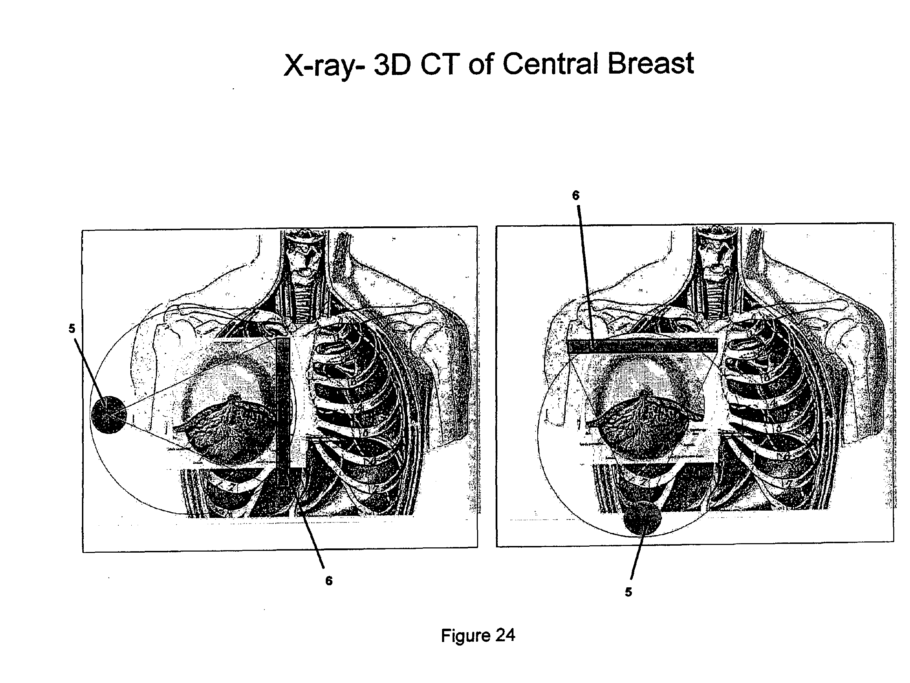

Referring now to Figure 24, x-ray source 5 and x-ray detector 6 are shown generating a fan/cone beam through patient's breast. Different views are shown to illustrate the positions of the x-ray source and detector around the patient's breast. The plurality of views allow reconstruction of x-ray views to form three dimensional tomographic slices of the breast's x-ray densities.

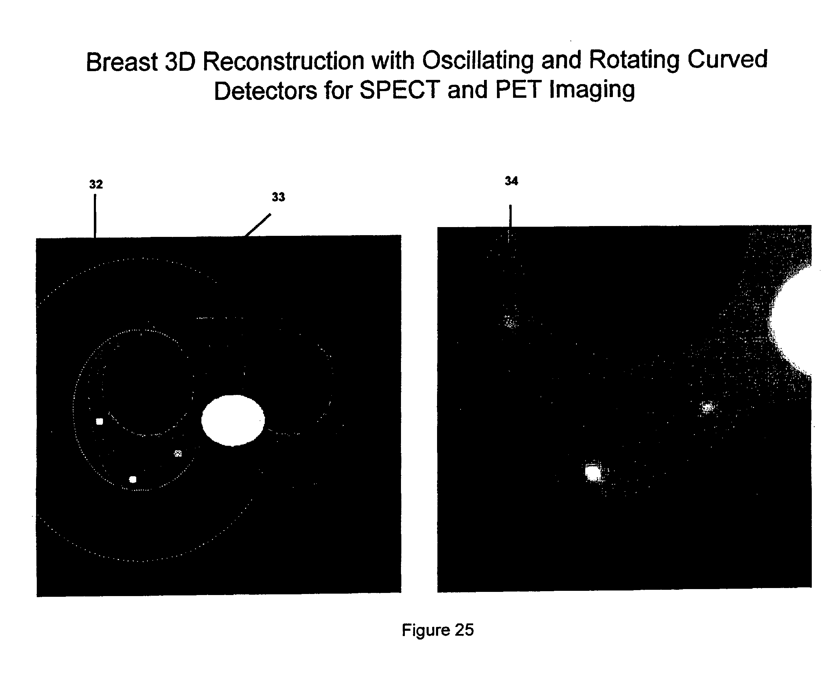

In Figure 25, reconstructed tomographic images are shown from the use of programmable detector orbits 32, oscillating curved gamma detector orbits 33 and reconstructed SPECT and PET images 34 from oscillating orbits. The programmable orbits are adjustable to patient's size and respective anatomy to obtain optimized spatial resolution and high sensitivity images of radioisotope distributions. Unique reconstruction tomographic processing is utilized to produce high quality imaging with these unique views in space.

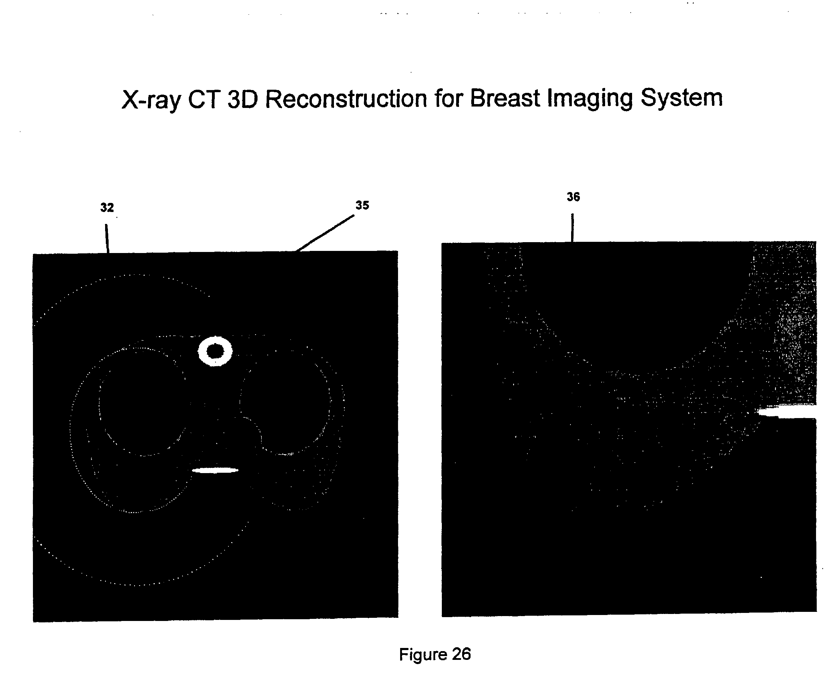

In Figure 26, reconstructed tomographic images are shown from the programmable detector orbits 32 and x-ray source and detector orbits 35 and reconstructed x-ray CT image from oscillating orbits 36. Here again, unique reconstruction tomographic processing is utilized to produce high quality imaging with these unique x-ray views in space.

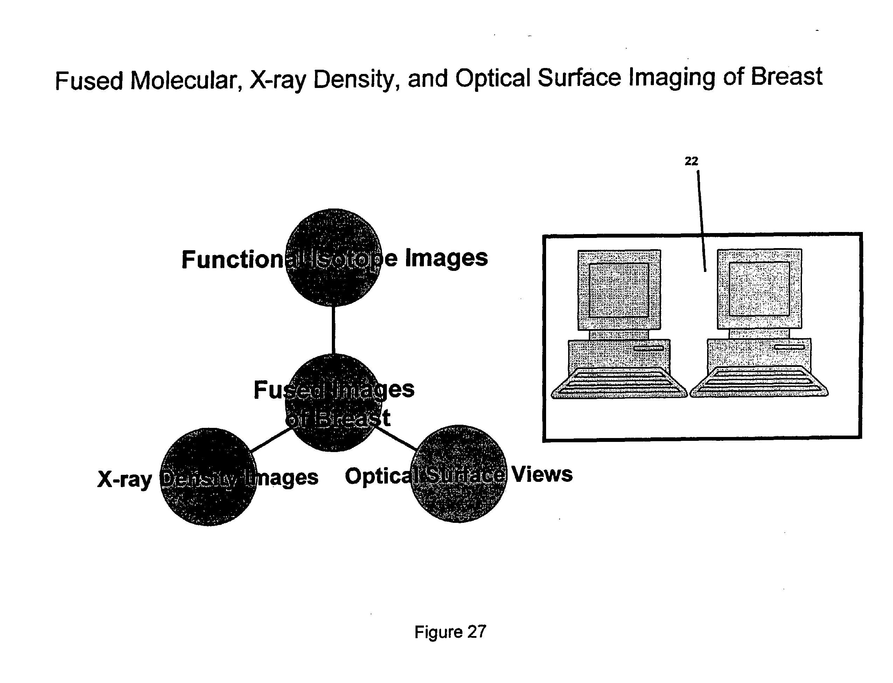

Referring now to Figure 27, the breast system display and analysis workstation 22 combines or fuses images. The radioisotope tomographic images from single gamma photon emitters with micro SPECT, positron emitters with coincident gamma rays for micro PET, combines with x-ray density images from x-ray micro CT and optical surface views for optical surface spectrums to form fused images of the breast.

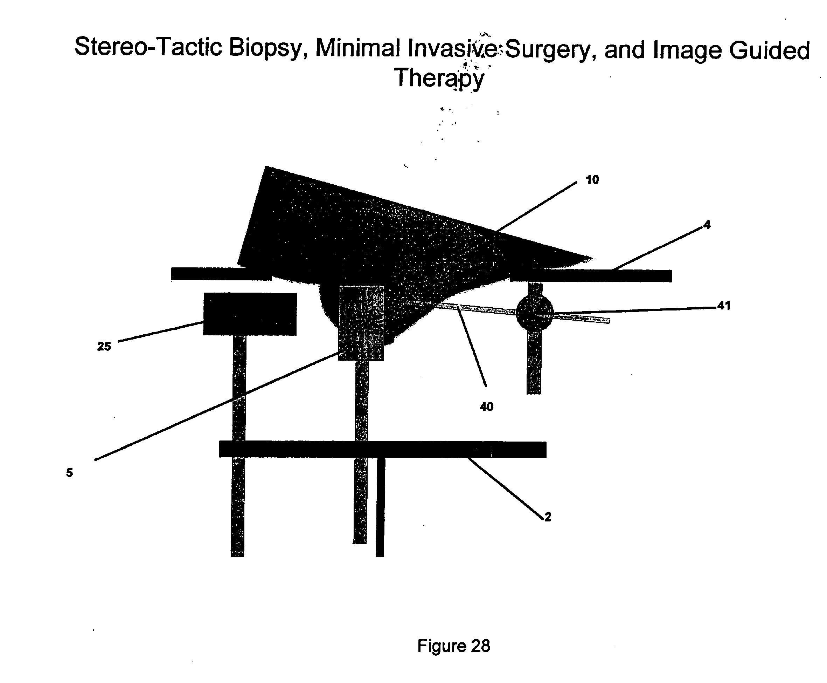

In Figure 28, a biopsy or surgical instrument 40 is shown being guided into the patient 10 and mechanically positioned with the stereo-tactic image guided holder 41. The breast system display and analysis workstation 22 generates interactive image guide information to align the stereo-tactic image guided holder 41 while patient 10 is lying prone and slightly tilted on breast imaging patient table 4. Also shown are the other basic multimodality imaging components of x-ray source 5, breast curved single photon and coincidence gamma detector 25, and rotate table 2 to generate images for biopsy, surgical removal, or therapy of breast cancer. The breast diagnostic apparatus for fused SPECT, PET, X-ray CT and Optical Surface Imaging of the breast described herein is a unique multimodality imaging device to uniquely scan the patient's entire breast for the presence of cancer.

Certain modifications and improvements will occur to those skilled in the art upon reading the foregoing. It is understood that all such modifications and improvements have been deleted herein for the sake of conciseness and readability, but are properly within the scope of the following claims.

## Claims

1. A multi-modality tomographic breast specific imaging system comprising at least one gamma ray detector for radioisotope tomography and means for performing x-ray computed tomography while the patient is lying in the prone position.

2. The imaging system as defined in claim 1 further including means for performing optical imaging, and optionally further including means for reconstructing radioisotope tomographic images produced by said at least one gamma ray detector, x-ray images produced by said x-ray computed tomography means and images produced by said optical imaging means, especially further including means for fusing said reconstructed radioisotopes tomographic images produced by said at least one gamma ray detector, said reconstructed images produced by x-ray computed tomography means and said reconstructed images produced by said optical imaging means, most especially wherein said fused images permit the stereo-tactic biopsy of the breast.

3. The imaging system as defined in claim 1 wherein said at least one gamma ray detector is positioned adjacent the central portion of the breast to produce images of the breast using single photon emission tomography, and optionally
a) further including means for positioning said at least one gamma ray detector with respect to the central portion of the breast, or
b) further including means for rotating said at least one gamma ray detector with respect to the central portion of the breast to produce images of the breast using single photon emission tomography, especially further including means for oscillating said at least one gamma ray detector with respect to the central portion of the breast to produce images of the breast using single photon emission tomography.

4. The imaging system as defined in claim 1 wherein said at least one gamma ray detector comprises oppositely disposed gamma ray detectors positioned adjacent the central portion of the breast to produce images of the breast using position emission tomography, and optionally
a) further including means for positioning said oppositely disposed gamma ray detectors with respect to the central portion of the breast, or
b) further including means for rotating said oppositely disposed gamma ray detectors with respect to the central portion of the breast to produce images of the breast using positron emission tomography, especially further including means for oscillating said oppositely disposed gamma ray detectors with respect to the central portion of the breast to produce images of the breast using positron emission tomography.

5. The imaging system as defined in claim 1 wherein said at least one gamma ray detector comprises a gamma ray detector positioned adjacent the upper outer quadrant of the breast to produce images of the breast using single photon emission tomography, and optionally
a) further including means for positioning said at least one gamma ray detector with respect to the upper outer quadrant of the breast, or
b) further including means for rotating said at least one gamma ray detector with respect to the upper outer quadrant of the breast to produce images of the breast using single photon emission tomography, especially further including means for oscillating said at least one gamma ray detector with respect to the upper outer quadrant of the breast to produce images of the breast using single photon emission tomography.

6. The imaging system as defined in claim 1 wherein said at least one gamma ray detector comprises oppositely disposed gamma ray detectors positioned adjacent the upper outer quadrant of the breast to produce images of the breast using positron emission tomography, and optionally
a) further including means for positioning said oppositely disposed gamma ray detectors with respect to the upper outer quadrant of the breast, or
b) further including means for rotating said oppositely disposed gamma ray detectors with respect of the upper outer quadrant of the breast to produce images of the breast using positron emission tomography, especially further including means for oscillating said oppositely disposed gamma ray detectors with respect to the upper outer quadrant of the breast to produce images of the breast using positron emission tomography.

7. The imaging system as defined in claim 1 wherein
a) said at least one gamma ray detector comprises oppositely disposed gamma ray detectors to produce images of the breast using single photon emission tomography and positron emission tomography, or
b) said at least one gamma ray detector is capable of producing images of the breast using both single photon emission tomography and positron emission tomography, or
c) said at least one gamma ray detector is curved in configuration.

8. The imaging system as defined in claim 1 wherein said x-ray computed tomography means comprises an x-ray source and said oppositely disposed x-ray detector, and optionally
a) further including means for positioning said x-ray source and said oppositely disposed x-ray detector with respect to the central portion of the breast, or
b) further including means for rotating said x-ray source and said oppositely disposed x-ray detector with respect to the central portion of the breast.

9. The imaging system as defined in claim 1 wherein said at least one gamma ray detector is comprised of a plurality of gamma ray detector modules, and optionally wherein each of said gamma ray detector modules is comprised of a collimation member, pixelated scintillation crystals, a photo-converter and an amplifier.

10. The imaging system as defined in claim 1 further including
a) a patient support member, said patient support member comprising a surface having at least one aperture therein to receive a breast of the patient permitting the breast to be unsupported during the imaging process, and optionally wherein said surface in said patient support member is configured so that the patient is lying in the prone position and to one side permitting a breast of the patient to be received within said at least one aperture in said patient support member for the imaging process, or
b) means for reconstructing radioisotope tomographic images produced by said at least one gamma ray detector and x-ray images produced by said x-ray computed tomography means, and optionally further including means for fusing said reconstructed radioisotopes tomographic images produced by said reconstructed images produced by at least one gamma ray detector and reconstructed images produced by said x-ray computed tomography means, especially wherein said fused images permit the stereo-tactic biopsy of the breast.
